# EUROPEAN PATENT SPECIFICATION

(11) **EP 1 648 465 B1**
(45) Date of publication and mention of the grant of the patent: **25.08.2010**
(21) Application number: 04743275.2
(22) Date of filing: 07.07.2004
(51) Int. Cl.: A61K 31/517, A61K 31/282, A61K 31/555, A61K 33/24, A61K 41/00, A61P 35/00

(54) **USE OF THE QUINAZOLINE DERIVATIVE ZD6474 COMBINED WITH PLATINUM COMPOUNDS AND OPTIONALLY IONISING RADIATION IN THE TREATMENT OF DISEASES ASSOCIATED WITH ANGIOGENESIS AND/OR INCREASED VASCULAR PERMEABILITY**
VERWENDUNG DES CHINAZOLIN-DERIVATS ZD6474 IN KOMBINATION MIT PLATINVERBINDUNGEN UND OPTIONAL IONISIERENDER STRAHLUNG BEI DER BEHANDLUNG VON ERKRANKUNGEN IM ZUSAMMENHANG MIT ANGIOGENESE UND/ODER ERHÖHTER GEFÄSSPERMEABILITÄT
UTILISATION DU DERIVE QUINAZOLINE ZD6474 EN COMBINAISON AVEC DES COMPOSES DE PLATINE ET EVENTUELLEMENT DE RAYONNEMENT IONISANT DANS LE TRAITEMENT DES MALADIES ASSOCIEES A L'ANGIOGENESE ET/OU A UNE PERMEABILITE VASCULAIRE ACCRUE

(30) Priority: 10.07.2003 GB 0316176; 24.03.2004 GB 0406546; 06.04.2004 GB 0407753
(43) Date of publication of application: 26.04.2006
(73) Proprietor: AstraZeneca AB, 151 85 Södertälje (SE)
(72) Inventor: WEDGE, Stephen Robert, AstraZeneca R & D Alderley, Macclesfield Cheshire SK10 4TG (GB); RYAN, Anderson Joseph, AstraZeneca R & D Alderley, Macclesfield Cheshire SK10 4TG (GB)
(86) International application number: PCT/GB2004/002932
(87) International publication number: WO 2005/004870

(56) References cited:
- WO-A-01/32651
- WO-A-98/13354
- GORSKI D H ET AL: "Blockade of the Vascular Endothelial Growth Factor Stress Response Increases the Antitumor Effects of Ionizing Radiation" CANCER RESEARCH, AMERICAN ASSOCIATION FOR CANCER RESEARCH, BALTIMORE, MD, US, vol. 59, 15 July 1999 (1999-07-15), pages 3374-3378, XP002256383 ISSN: 0008-5472

## Description

The present invention relates to a pharmaceutical composition comprising ZD6474 and a platinum anti-tumour agent; to a combination product comprising ZD6474 and a platinum anti-tumour agent for use in a method of treatment of a human or animal body by therapy; to a kit comprising ZD6474 and a platinum anti-tumour agent: to the use of ZD6474 and a platinum anti-tumour agent in the manufacture of a medicament for use in the production of an antiangiogenic and/or vascular permeability reducing effect in a warm-blooded animal such as a human which is optionally being treated with ionising radiation.

Normal angiogenesis plays an important role in a variety of processes including embryonic development, wound healing and several components of female reproductive function. Undesirable or pathological angiogenesis has been associated with disease states including diabetic retinopathy, psoriasis, cancer, rheumatoid arthritis, atheroma, Kaposi's sarcoma and haemangioma (Fan et al, 1995, Trends Pharmacol. Sci. 16: 57-66; Folkman, 1995, Nature Medicine 1: 27-31). Alteration of vascular permeability is thought to play a role in both normal and pathological physiological processes (Cullinan-Bove et al, 1993, Endocrinology 133: 829-837; Senger et al., 1993, Cancer and Metastasis Reviews, 12: 303-324). Several polypeptides with *in vitro* endothelial cell growth promoting activity have been identified including, acidic and basic fibroblast growth factors (aFGF & bFGF) and vascular endothelial growth factor (VEGF). By virtue of the restricted expression of its receptors, the growth factor activity of VEGF, in contrast to that of the PGFs, is relatively specific towards endothelial cells. Recent evidence indicates that VEGF is an important stimulator of both normal and pathological angiogenesis (Jakeman et al, 1993, Endocrinology, 133: 848-859; Kolch et al, 1995, Breast Cancer Research and Treatment. 36:139-155) and vascular permeability (Connolly et al, 1989, J. Biol. Chem. 264: 20017-20024). Antagonism of VEGF action by sequestration of VEGF with antibody can result in inhibition of tumour growth (Kim et al, 1993, Nature 362: 841-844).

Preceptor tyrosine kinases (RTKs) are important in the transmission of biochemical signals across the plasma membrane of cells. These transmembrane molecules characteristically consist of an extracellular ligand-binding domain connected through a segment in the plasma membrane to an intracellular tyrosine kinase domain. Binding of ligand to the receptor results in stimulation of the receptor-associated tyrosine kinase activity which leads to phosphorylation of tyrosine residues on both the receptor and other intracellular molecules. These changes in tyrosine phosphorylation initiate a signalling cascade leading to a variety of cellular responses. To date, at least nineteen distinct RTK subfamilies, defined by amino acid sequence homology, have been identified. One of these subfamilies is presently comprised by the fms-like tyrosine kinase receptor, Flt-1 (also referred to as VEGFR-1), the kinase insert domain-containing receptor, KDR (also referred to as VEGFR-2 or Flk-1), and another fms-like tyrosine kinase receptor, Flt-4. Two of these related RTKs, Flt-1 and KDR, have been shown to bind VEGF with high affinity (De Vries et al, 1992, Science 255: 989-991; Terman et al, 1992, Biochem. Biophys. Res. Comm. 1992, 187: 1579-1586). Binding of VEGF to these receptors expressed in heterologous cells has been associated with changes in the tyrosine phosphorylation status of cellular proteins and calcium fluxes.

VEGF is a key stimulus for vasculogenesis and angiogenesis. This cytokine induces a vascular sprouting phenotype by inducing endothelial cell proliferation, protease expression and migration, and subsequent organisation of cells to form a capillary tube (Keck, P.J., Hauser, S.D., Krivi, G., Sanzo, K., Warren, T., Feder, J., and Connolly, D.T., Science (Washington DC), 246: 1309-1312, 1989; Lamoreaux, W.J., Fitzgerald, M.E., Reiner, A., Hasty, K.A., and Charles, S.T., Microvasc. Res., 55: 29-42, 1998; Pepper, M.S., Montesano, R., Mandroita, S.J., Orci, L. and Vassalli, J.D., Enzyme Protein, 49: 138-162, 1996.). In addition, VEGF induces significant vascular permeability (Dvorak, H.F., Detmar, M., Claffey, K.P., Nagy, J.A., van de Water, L., and Senger, D.R., (Int. Arch. Allergy Immunol., 107: 233-235, 1995; Bates, D.O., Heald, R.I., Curry, F.E. and Williams, B. J. Physiol. (Lond.), 533: 263-272, 2001), promoting formation of a hyper-permeable, immature vascular network which is characteristic of pathological angiogenesis.
It has been shown that activation of KDR alone is sufficient to promote all of the major phenotypic responses to VEGF, including endothelial cell proliferation, migration, and survival, and the induction of vascular permeability (Meyer, M., Clauss, M., Lepple-Wienhues, A., Waltenberger, J., Augustin, H.G., Ziche, M., Lanz, C., Büttner, M., Rziha, H-J., and Dehio, C., EMBO J., 18: 363-374, 1999; Zeng, H., Sanyal, S. and Mukhopadhyay, D., J. Biol. Chem., 276: 32714-32719, 2001; Gille, H., Kowalski, J., Li, B., LeCouter, J., Moffat, B, Zioncheck, T.F., Pelletier, N. and Ferrara, N., J. Biol. Chem., 276: 3222-3230, 2001).

Quinazoline derivatives which are inhibitors of VEGF receptor tyrosine kinase are described in International Patent Applications Publication Nos. WO 98/13354 and WO 01/32651. In WO 98/13354 and WO 01/32651 compounds are described which possess activity against VEGF receptor tyrosine kinase (VEGF RTK) whilst possessing some activity against epidermal growth factor (EGF) receptor tyrosine kinase (EGF RTK).
ZD6474 is 4-(4-bromo-2-fluoroanilino)-6-methoxy-7-(1-methylpiperidin-4-ylmethoxy)quinazoline:

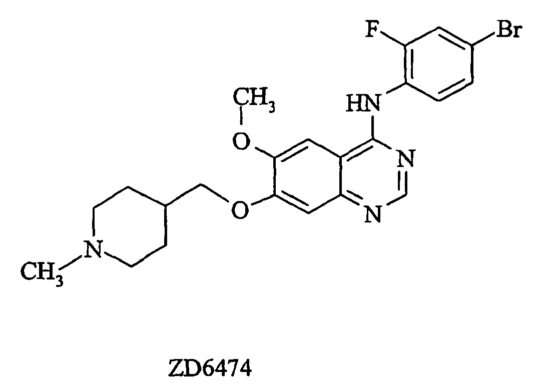

ZD6474 falls within the broad general disclosure of WO 98/13354 and is exemplified in WO 01/32651. ZD6474 is a potent inhibitor of VEGF RTK and also has some activity against EGF RTK. ZD6474 has been shown to elicit broad-spectrum anti-tumour activity in a range of models following once-daily oral administration (Wedge S.R., Ogilvie D.J., Dukes M. et al, Proc. Am. Assoc. Canc. Res. 2001; 42: abstract 3126).

In WO 98/13354 and WO 01/32651 it is stated that compounds of their inventions: "may be applied as a sole therapy or may involve, in addition to a compound of the invention, one or more other substances and/or treatments. Such conjoint treatment may be achieved by way of the simultaneous, sequential or separate administration of the individual components of the treatment."
WO 98/13354 and WO 01/32651 then go on to describe examples of such conjoint treatment including surgery, radiotherapy and various types of chemotherapeutic agent.

Nowhere in WO 98/13354 and WO 01/32651 is the specific combination of ZD6474 and a platinum anti-tumour agent suggested.

Nowhere in WO 98/13354 and WO 01/32651 does it state that use of any compound of the invention therein with other treatments will produce surprisingly beneficial effects.

Unexpectedly and surprisingly we have now found that the particular compound ZD6474 used in combination with a particular selection from the broad description of combination therapies listed in WO 98/13354 and WO 01/32651, namely with a platinum anti-tumour agent, produces significantly better effects than any one of ZD6474 and a platinum anti-tumour agent used alone. In particular, ZD6474 used in combination with a platinum anti-tumour agent produces significantly better effects on solid tumours than any one of ZD6474 and a platinum anti-tumour agent used alone.

A platinum anti-tumour agent is any anti-tumour agent containing platinum. Platinum anti-tumour agents include cisplatin, carboplatin, oxaliplatin, nedaplatin, lobaplatin, satraplatin and AMD473.

Anti-cancer effects of a method of treatment of the present invention include, but are not limited to, anti-tumour effects, the response rate, the time to disease progression and the survival rate. Anti-tumour effects of a method of treatment of the present invention include but are not limited to, inhihition of tumour growth, tumour growth delay, reg-ression of tumour, shrinkage of tumour, increased time to regrowth of tumour on cessation of treatment, slowing of disease progression. It is expected that when a combination of the present invention is administered to a warm-blooded animal such as a human, in need of treatment for cancer, with or without a solid tumour, will produce an effect, as measured by, for example, one or more of: the extent of the anti-tumour effect, the response rate, the time to disease progression and the survival race. Anti-cancer effects include prophylactic treatment as well as treatment of existing disease.
According to the present invention there is provided a pharmaceutical composition which comprises ZD6474 or a pharmaceutically acceptable salt thereof, and a platinum anti-tumour agent, in association with a pharmaceutically acceptable excipient or carrier.

According to a further aspect of the present invention there is provided a combination product comprising ZD6474 or a pharmaceutically acceptable salt thereof and a platinum anti-tumour agent, for use in a method of treatment of a human or animal body by therapy.

According to a further aspect of the present invention there is provided a kit comprising ZD6474 or a pharmaceutically acceptable salt thereof, and a platinum anti-tumour agen t.

According to a further aspect of the present invention there is provided a kit comprising:
a) ZD6474 or a pharmaceutically acceptable salt thereof in a first unit dosage form;
b) a platinum anti-tumour agent in a second unit dosage form; and
c) container means for containing said first and second dosage forms.

According to a further aspect of the present invention there is provided a kit comprising:
a) ZD6474 or a pharmaceutically acceptable salt thereof, together with a pharmaceutically acceptable excipient or carrier, in a first unit dosage form;
b) a platinum anti-tumour agent together with a pharmaceutically acceptable excipient or carrier, in a second unit dosage form; and
c) container means for containing said first and second dosage forms.

According to a further aspect of the present invention there is provided the use of ZD6474 or a pharmaceutically acceptable salt thereof and a platinum anti-tumour agent in the manufacture of a medicament for use in the production of an antiangiogenic and/or vascular permeability reducing effect in a warm-blooded animal such as a human.

According to a further aspect of the present invention there is provided the use of ZD6474 or a pharmaceutically acceptable salt thereof and a platinum anti-tumour agent in the manufacture of a medicament for use in the production of an anti-cancer effect in a warm-blooded animal such as a human.

According to a further aspect of the present invention there is provided the use of ZD6474 or a pharmaceutically acceptable salt thereof and a platinum anti-tumour agent in the manufacture of a medicament for use in the production of an anti-tumour effect in a warm-blooded animal such as a human.

According to a further aspect of the present invention there is provided a therapeutic combination use comprising the administration of an effective amount of ZD6474 or a pharmaceutically acceptable salt thereof, optionally together with a pharmaceutically acceptable excipient or carrier, and the simultaneous, sequential or separate administration of an effective amount of a platinum anti-tumour agent, wherein a platinum anti-tumour agent may optionally be administered together with a pharmaceutically acceptable excipient or carrier, to a warm-blooded animal such as a human in need of such therapeutic treatment. Such therapeutic use includes an antiangiogenic and/or vascular permeability effect, an anti-cancer effect and an anti-tumour effect.

A combination use of the present invention as defined herein may be achieved by way of the simultaneous, sequential or separate administration of the individual components of said treatment. A combination use as defined herein may be applied as a sole therapy or may involve surgery or radiotherapy or an additional chemotherapeutic agent in addition to a combination treatment of the invention.

Surgery may comprise the step of partial or complete tumour resection, prior to, during or after the administration of the combination treatment with ZD6474 described herein.

Other chemotherapeutic agents for optional use with a combination treatment of the present invention include those described in WO 01/32651.

Such chemotherapy may cover five main categories of therapeutic agent:
(i) other antiangiogenic agents including vascular targeting agents;
(ii) cytostatic agents;
(iii) biological response modifiers (for example interferon);
(iv) antibodies (for example edrecolomab); and
(v) antiproliferative/antineoplastic drugs and combinations thereof, as used in medical oncology; and other categories of agent are:
(vi) antisense therapies;
(vii) gene therapy approaches; and
(ix) immunotherapy approaches.

Particular examples of chemotherapeutic agents for use with a combination treatment of the present invention are raltitrexed, etoposide, vinorelbine, paclitaxel, docetaxel, gemcitabine, irinotecan (CPT-11) and 5-fluorouracil (5-FU); such combinations are expected to be particularly useful for the treatment of cancer of the lung, head and neck, colon, rectum, oesophagus, stomach, cervix, ovary, skin, breast, bladder and pancreas.

The administration of a triple combination of ZD6474, a platinum anti-tumour agent and ionising radiation may produce effects, such as anti-tumour effects, greater than those achieved with any of ZD6474, a platinum anti-tumour agent and ionising radiation used alone, greater than those achieved with the combination of ZD6474 and a platinum anti-tumour agent, greater than those achieved with the combination of ZD6474 and ionising radiation, greater than those achieve with the combination of a platinum anti-tumour agent and ionising radiation.

According to a further aspect of the present invention there is provided the use of ZD6474 or a pharmaceutically acceptable salt thereof and a platinum anti-tumour agent in the manufacture of a medicament for use in the production of an antiangiogenic and/or vascular permeability reducing effect in a warm-blooded animal such as a human which is being treated with ionising radiation.

According to a further aspect of the present invention there is provided the use of ZD6474 or a pharmaceutically acceptable salt thereof and a platinum anti-tumour agent in the manufacture of a medicament for use in the production of an anti-cancer effect in a warm-blooded animal such as a human which is being treated with ionising radiation.

According to a further aspect of the present invention there is provided the use of ZD6474 or a pharmaceutically acceptable salt thereof and a platinum anti-tumour agent in the manufacture of a medicament for use in the production of an anti-tumour effect in a warm-blooded animal such as a human which is being treated with ionising radiation.

According to a further aspect of the present invention there is provided a therapeutic combination use comprising the administration of an effective amount of ZD6474 or a pharmaceutically acceptable salt thereof, optionally together with a pharmaceutically acceptable excipient or carrier, and the administration of an effective amount of a platinum anti-tumour agent, optionally together with a pharmaceutically acceptable excipient or carrier and the administration of an effective amount of ionising radiation, to a warm-blooded animal such as a human in need of such therapeutic treatment wherein the ZD6474, a platinum anti-tumour agent and ionising radiation may be administered simultaneously, sequentially or separately and in any order.

A warm-blooded animal such as a human which is being treated with ionising radiation means a warm-blooded animal such as a human which is treated with ionising radiation before, after or at the same time as the administration of a medicament or combination treatment comprising ZD6474 and a platinum anti-tumour agent. For example said ionising radiation may be given to said warm-blooded animal such as a human within the period of a week before to a week after the administration of a medicament or combination treatment comprising ZD6474 and a platinum anti-tumour agent. This means that ZD6474, a platinum anti-tumour agent and ionising radiation may be administered separately or sequentially in any order, or may be administered simultaneously. The warm-blooded animal may experience the effect of each of ZD6474, a platinum anti-tumour agent and radiation simultaneously.

According to one aspect of the present invention the ionising radiation is administered before one of ZD6474 and a platinum anti-tumour agent or after one of ZD6474 and a platinum anti-tumour agent.

According to one aspect of the present invention the ionising radiation is administered before both ZD6474 and a platinum anti-tumour agent or after both ZD6474 and a platinum anti-tumour agent.

According to one aspect of the present invention ZD6474 is administered to a warm-blooded animal after the animal has been treated with ionising radiation.

According to another aspect of the present invention the effect of a use of the present invention is expected to be at least equivalent to the addition of the effects of each of the components of said treatment used alone, that is, of each of ZD6474 and a platinum anti-tumour agent used alone or of each of ZD6474, a platinum anti-tumour agent and ionising radiation used alone.

According to another aspect of the present invention the effect of a use of the present invention is expected to be greater than the addition of the effects of each of the components of said treatment used alone, that is, of each of ZD6474 and a platinum anti-tumour agent used alone or of each of ZD6474, a platinum anti-tumour agent and ionising radiation used alone.

According to another aspect of the present invention the effect of a use of the present invention is expected to be a synergistic effect.

According to the present invention a combination treatment is defined as affording a synergistic effect if the effect is therapeutically superior, as measured by, for example, the extent of the response, the response rate, the time to disease progression or the survival period, to that achievable on dosing one or other of the components of the combination treatment at its conventional dose. For example, the effect of the combination treatment is synergistic if the effect is therapeutically superior to the effect achievable with ZD6474 or a platinum anti-tumour agent or ionising radiation alone. Further, the effect of the combination treatment is synergistic if a beneficial effect is obtained in a group of patients that does not respond (or responds poorly) to ZD6474 or a platinum anti-tumour agent or ionising radiation alone. In addition, the effect of the combination treatment is defined as affording a synergistic effect if one of the components is dosed at its conventional dose and the other component(s) is/are dosed at a reduced dose and the therapeutic effect, as measured by, for example, the extent of the response, the response rate, the time to disease progression or the survival period, is equivalent to that achievable on dosing conventional amounts of the components of the combination treatment, In particular, synergy is deemed to be present if the conventional dose of ZD6474 or a platinum anti-tumour agent or ionising radiation may be reduced without detriment to one or more of the extent of the response, the response rate, the time to disease progression and survival data, in particular without detriment to the duration of the response, but with fewer and/or less troublesome side-effects than those that occur when conventional doses of each component are used.

As stated above the combination uses of the present invention as defined herein are of interest for their antiangiogenic and/or vascular permeability effects. Angiogenesis and/or an increase in vascular permeability is present in a wide range of disease states including cancer (including leukaemia, multiple myeloma and lymphoma), diabetes, psoriasis, rheumatoid arthritis, Kaposi's sarcoma, haemangioma, acute and chronic nephropathies, atheroma, arterial restenosis, autoimmune diseases, acute inflammation, lymphoedema, endometriosis, dysfunctional uterine bleeding and ocular diseases with retinal vessel proliferation including age-related macular degeneration. Combination uses of the present invention are expected to be particularly useful in the prophylaxis and treatment of diseases such as cancer and Kaposi's sarcoma. In particular such combination uses of the invention are expected to slow advantageously the growth of primary and recurrent solid tumours of, for example, the colon, pancreas, bladder, breast, prostate, lungs and skin. More especially combination treatments of the present invention are expected to slow advantageously the growth of tumours in colorectal cancer and in lung cancer, for example mesothelioma and non-small cell lung cancer (NSCLC). More particularly such combination uses of the invention are expected to inhibit any form of cancer associated with VEGF including leukaemia, mulitple myeloma and lymphoma and also, for example, to inhibit the growth of those primary and recurrent solid tumours which are associated with VEGF, especially those tumours which are significantly dependent on VEGF for their growth and spread, including for example, certain tumours of the colon (including rectum), pancreas, bladder, breast, prostate, lung, vulva, skin and particularly NSCLC.

In another aspect of the present invention ZD6474 and a platinum anti-tumour agent, optionally with ionising radiation, are expected to inhibit the growth of those primary and recurrent solid tumours which are associated with VEGF especially those tumours which are significantly dependent on VEGF for their growth and spread.

In another aspect of the present invention ZD6474 and a platinum anti-tumour agent, optionally with ionising radiation, are expected to inhibit the growth of those primary and recurrent solid tumours which are associated with both VEGF and EGF especially those tumours which are significantly dependent on VEGF and EGF for their growth and spread.

The compositions described herein may be in a form suitable for oral administration, for example as a tablet or capsule, for nasal administration or administration by inhalation, for example as a powder or solution, for parenteral injection (including intravenous, subcutaneous, intramuscular, intravascular or infusion) for example as a sterile solution, suspension or emulsion, for topical administration for example as an ointment or cream, for rectal administration for example as a suppository or the route of administration may be by direct injection into the tumour or by regional delivery or by local delivery. In other embodiments of the present invention the ZD6474 of the combination treatment may be delivered endoscopically, intratracheally, intralesionally, percutaneously, intravenously, subcutaneously, intraperitoneally or intratumourally. Preferably ZD6474 is administered orally. In general the compositions described herein may be prepared in a conventional manner using conventional excipients. The compositions of the present invention are advantageously presented in unit dosage form.

ZD6474 will normally be administered to a warm-blooded animal at a unit dose within the range 10-500mag per square metre body area of the animal, for example approximately 0.3-15mg/kg in a human. A unit dose in the range, for example, 0.3-15mg/kg, preferably 0.5-5mg/kg is envisaged and this is normally a therapeutically-effective dose. A unit dosage form such as a tablet or capsule will usually contain, for example 25-500mg of active ingredient. Preferably a daily dose in the range of 0.5-5mg/kg is employed.

Platinum anti-tumour agents may be dosed according to known routes of administration and dosages.

For example cisplatin may be administered as a single intravenous infusion over a period of 6-8 hours at a dose of 40-120mg/m² every 3-4 weeks. Alternatively for example cisplatin may be administered as a single intravenous infusion over a period of 6-8 hours at a dose of 15-20mg/m² daily for up to 5 days every 3-4 weeks.

For example carboplatin may be administered as a single short-term intravenous infusion over a period of 15-60 minutes at a dose of 250-400mg/M² every 4 weeks.

For example oxaliplatin may be administered by intravenous infusion over a period of 2-6 hours at a dose of about 85mg/m² every 2 weeks.
The dosages and schedules may vary according to the particular disease state and the overall condition of the patient. Dosages and schedules may also vary if, in addition to a combination treatment of the present invention, one or more additional chemotherapeutic agents is/are used. Scheduling can be determined by the practitioner who is treating any particular patient.

Radiotherapy may be administered according to the known practices in clinical radiotherapy. The dosages of ionising radiation will be those known for use in clinical radiotherapy. The radiation therapy used will include for example the use of y-rays, X-rays, and/or the directed delivery of radiation from radioisotopes. Other forms of DNA damaging factors are also included in the present invention such as microwaves and UV-irradiation. For example X-rays may be dosed in daily doses of 1.8-2.0Gy, 5 days a week for 5-6 weeks. Normally a total fractionate dose will lie in the range 45-60Gy. Single larger doses, for example 5-10Gy may be administered as part of a course of radiotherapy. Single doses may be administered intraoperatively. Hyperfractionated radiotherapy may be used whereby small doses of X-rays are administered regularly over a period of time, for example 0.1Gy per hour over a number of days. Dosage ranges for radioisotopes vary widely, and depend on the half-life of the isotope, the strength and type of radiation emitted, and on the uptake by cells.

As stated above the size of the dose of each therapy which is required for the therapeutic or prophylactic treatment of a particular disease state will necessarily be varied depending on the host treated, the route of administration and the severity of the illness being treated. Accordingly the optimum dosage may be determined by the practitioner who is treating any particular patient. For example, it may be necessary or desirable to reduce the above-mentioned doses of the components of the combination treatments in order to reduce toxicity.

The present invention relates to combinations of a platinum anti-tumour agent with ZD6474 or with a salt of ZD6474.

Salts of ZD6474 for use in pharmaceutical compositions will be pharmaceutically acceptable salts, but other salts may be useful in the production of ZD6474 and its pharmaceutically acceptable salts. Such salts may be formed with an inorganic or organic base which affords a pharmaceutically acceptable cation. Such salts with inorganic or organic bases include for example an alkali metal salt, such as a sodium or potassium salt, an alkaline earth metal salt such as a calcium or magnesium salt, an ammonium salt or for example a salt with methylamine, dimethylamine, trimethylamine, piperidine, morpholine or tris-(2-hydroxyethyl)amine.

ZD6474 may be synthesised according to any of the known processes for making ZD6474. For example ZD6474 may be made according to any of the processes described in WO 01/32651; for example those described in Examples 2(a), 2(b) and 2(c) of WO 01/32651.

Platinum anti-tumour agents are commercially available.

The following tests may be used to demonstrate the activity of ZD6474 in combination with a platinum anti-tumour agent.

### Calu 6 lung cancer xenograft model

A human lung cancer (NSCLC) xenograft model is used. Athymic nude mice are injected subcutaneously (s.c.) with Calu 6 human tumour cells. Treatment begins after 7-10 days (in a particular experiment 13 days) when tumours are established (tumour volume 100-300mm³, in a particular experiment = 200 mm³). Groups of animals (n = 8 per group in a particular experiment but could be 10-12 per group) are randomized to receive a single treatment with cisplatin (4 mg/kg intraperitoneally (i.p.)) on day of randomization, or treatment with ZD6474 (25 - 75 mg/m² orally (p.o.) daily, or 6.25 - 25mg/kg p.o. daily, in a particular experiment 25mg/kg) for the duration of the experiment, or drug vehicles only. An additional group of animals (n = 8 in a particular experiment but could be 10-12) receives a combination of cisplatin and ZD6474, using the same doses and schedules as used for single agent treatment. On days where animals received both ZD6474 and cisplatin the cisplatin was administered 2 hours after oral dosing with ZD6474.

Animals in all groups are sacrificed when the control tumours reach approximately 2.0 cm³ or alternatively on the basis of a certain number of doses of treatment received. Tumour size is assessed throughout the experiment by using caliper measurements. Antitumour effects are determined by comparing tumour growth in the drug-treated groups with tumour growth in the vehicle treated groups. Additionally, the effects of combination treatment are assessed by comparing tumour growth in the group of animals receiving cisplatin plus ZD6474 with the tumour growth in the groups where animals received single agent therapy alone.

Statistical significance was evaluated using a one-tailed two-sample t-test.

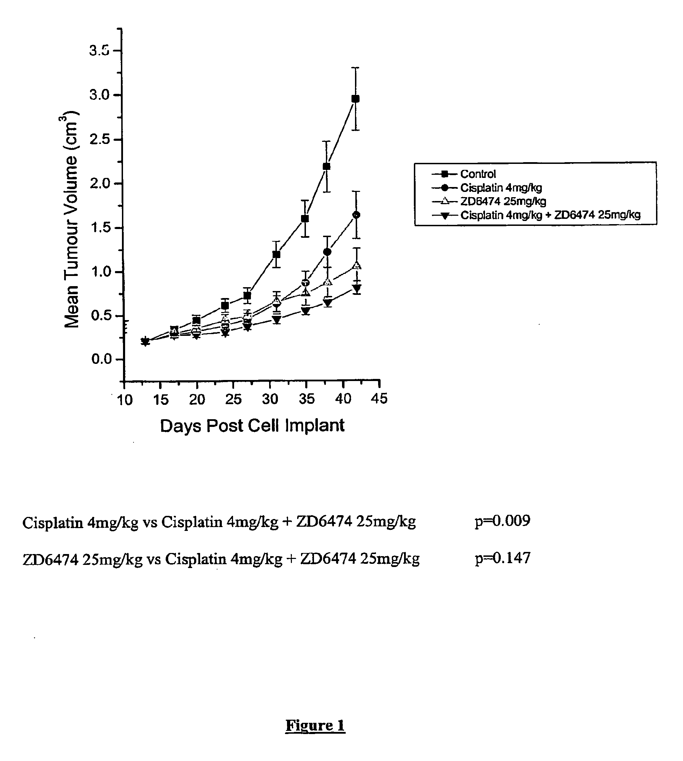

The results using cisplatin (4mg/kg) and ZD6474 (25mg/kg) are shown in Figure 1.

The growth of the tumours was inhibited significantly more by the combination of the two agents ZD6474 (25mg/kg) and cisplatin (4mg/kg) than by cisplatin alone. The effect of the combination was also greater than that of ZD6474 alone.

An analogous experiment may be used to look at the combination of ZD6474 and a platinum anti-tumour agent with ionising radiation.

## Claims

1. Use of 4-(4-bromo-2-fluoroanilino)-6-methoxy-7-(1-memylpipendin-4-ylmethoxy)quinazoline or a pharmaceutically acceptable salt thereof and a platinum anti-tumour agent in the manufacture of a medicament for use in the production of an antiangiogenic and/or vascular permeability reducing effect in a warm-blooded animal such as a human.

2. Use of 4-(4-bromo-2-fluoroanilino)-6-methoxy-7-(1-methylpipendin-4-ylmethoxy)quinazoline or a pharmaceutically acceptable salt thereof and a platinum anti-tumour agent in the manufacture of a medicament for use in the production of an anti-cancer effect in a warm-blooded animal such as a human.

3. Use of 4-(4-bromo-2-fluoroanilino)-6-methoxy 7-(1-methylpiperidin-4-ylmethoxy)quinazoline or a pharmaceutically acceptable salt thereof and a platinum anti-tumour agent in the manufacture of a medicament for use in the production of an anti-tumour effect in a warm-blooded animal such as a human.

4. Use of 4-(4-bromo-2-fluoroanilino)-6-methoxy-7-(1-methylpiperidin-4-ylmethoxy)quinazoline or a pharmaceutically acceptable salt thereof and a platinum anti-tumour agent in the manufacture of a medicament for use in the production of an antiangiogenic and/or vascular permeability reducing effect in a warm-blooded animal such as a human which is being treated with ionising radiation.

5. Use of 4-(4-bromo-2-fluoroanilino)-6-methoxy-7-(1-methylpiperidin-4-ylmethoxy)quinazoline or a pharmaceutically acceptable salt thereof and a platinum anti-tumour agent in the manufacture of a medicament for use in the production of an anti-cancer effect in a warm-blooded animal such as a human which is being treated with ionising radiation.

6. Use of 4-(4-bromo-2-fluoroadmo)-6-methoxy-7-(1-methylpipeidin-4-ylmethoxy)quinazoline or a pharmaceutically acceptable salt thereof and a platinum anti-tumour agent in the manufacture of a medicament for use in the production of an anti-tumour effect in a warm-blooded animal such as a human which is being treated with ionising radiation.

7. Use according to claim 2 or claim 5 wherein the cancer is non-small cell lung cancer (NSCLC).

8. Use according to claim 3 or claim 6 wherein the tumour is a tumour of the colon (including rectum), pancreas, bladder, breast, prostate, lung, vulva or skin.

9. Use according to any one of claims 1-8 wherein the platinum anti-tumour agent is cisplatin.

10. Use according to any one of claims 1-8 wherein the platinum anti-tumour agent is carboplatin.

11. Use according to any one of claims 1-8 wherein the platinum anti-tumour agent is oxaliplatin.

12. A pharmaceutical composition which comprises 4-(4-bromo-2-fluoroanilino)-6-methoxy-7-(1-methylpiperidin-4-ylmethoxy)quinazoline or a pharmaceutically acceptable salt thereof, and a platinum anti-tumour agent, in association with a pharmaceutically acceptable excipient or carrier.

13. A kit comprising 4-(4-bromo-2-fluoroanilino)-6-methoxy-7-(1-methylpiperidin-4-ylmethoxy)quinazoline or a pharmaceutically acceptable salt thereof, and a platinum anti-tumour agent.

## Patentansprüche

1. Verwendung von 4-(4-Brom-2-fluoranilino)-6-methoxy-7-(1-methylpiperidin-4-ylmethoxy)-chinazolin oder eines pharmazeutisch annehmbaren Salzes davon und eines Platin-Antitumormittels bei der Herstellung eines Arzneimittels zur Verwendung bei der Erzielung einer antiangiogenen und/oder gefäßpermeabilitätssenkenden Wirkung bei einem Warmblüter wie einem Menschen.

2. Verwendung von 4-(4-Brom-2-fluoranilino)-6-methoxy-7-(1-methylpiperidin-4-ylmethoxy)-chinazolin oder eines pharmazeutisch annehmbaren Salzes davon und eines Platin-Antitumormittels bei der Herstellung eines Arzneimittels zur Verwendung bei der Erzielung einer Antikrebswirkung bei einem Warmblüter wie einem Menschen.

3. Verwendung von 4-(4-Brom-2-fluoranilino)-6-methoxy-7-(1-methylpiperidin-4-ylmethoxy)-chinazolin oder eines pharmazeutisch annehmbaren Salzes davon und eines Platin-Antitumormittels bei der Herstellung eines Arzneimittels zur Verwendung bei der Erzielung einer Antitumorwirkung bei einem Warmblüter wie einem Menschen.

4. Verwendung von 4-(4-Brom-2-fluoranilino)-6-methoxy-7-(1-methylpiperidin-4-ylmethoxy)-chinazolin oder eines pharmazeutisch annehmbaren Salzes davon und eines Platin-Antitumormittels bei der Herstellung eines Arzneimittels zur Verwendung bei der Erzielung einer antiangiogenen und/oder gefäßpermeabilitätssenkenden Wirkung bei einem Warmblüter wie einem Menschen, der mit ionisierender Strahlung behandelt wird.

5. Verwendung von 4-(4-Brom-2-fluoranilino)-6-methoxy-7-(1-methylpiperidin-4-ylmethoxy)-chinazolin oder eines pharmazeutisch annehmbaren Salzes davon und eines Platin-Antitumormittels bei der Herstellung eines Arzneimittels zur Verwendung bei der Erzielung einer Antikrebswirkung bei einem Warmblüter wie einem Menschen, der mit ionisierender Strahlung behandelt wird.

6. Verwendung von 4-(4-Brom-2-fluoranilino)-6-methoxy-7-(1-methylpiperidin-4-ylmethoxy)-chinazolin oder eines pharmazeutisch annehmbaren Salzes davon und eines Platin-Antitumormittels bei der Herstellung eines Arzneimittels zur Verwendung bei der Erzielung einer Antitumorwirkung bei einem Warmblüter wie einem Menschen, der mit ionisierender Strahlung behandelt wird.

7. Verwendung nach Anspruch 2 oder 5, bei der es sich bei dem Krebs um nicht-kleinzelliges Bronchialkarzinom (NSCLC) handelt.

8. Verwendung nach Anspruch 3 oder 6, bei der es sich bei dem Tumor um einen Tumor des Kolons (einschließlich des Rektums), der Bauchspeicheldrüse, der Blase, der Brust, der Prostata, der Lunge, der Vulva oder der Haut handelt.

9. Verwendung nach einem der Ansprüche 1-8, bei der es sich bei dem Platin-Antitumormittel um Cisplatin handelt.

10. Verwendung nach einem der Ansprüche 1-8, bei der es sich bei dem Platin-Antitumormittel um Carboplatin handelt.

11. Verwendung nach einem der Ansprüche 1-8, bei der es sich bei dem Platin-Antitumormittel um Oxaliplatin handelt.

12. Pharmazeutische Zusammensetzung, die 4-(4-Brom-2-fluoranilino)-6-methoxy-7-(1-methylpiperidin-4-ylmethoxy)chinazolin oder ein pharmazeutisch annehmbares Salz davon und ein Platin-Antitumormittel zusammen mit einem pharmazeutisch annehmbaren Hilfsstoff oder Träger enthält.

13. Kit, enthaltend 4-(4-Brom-2-fluoranilino)-6-methoxy-7-(1-methylpiperidin-4-ylmethoxy)-chinazolin oder ein pharmazeutisch annehmbares Salz davon und ein Platin-Antitumormittel.

## Revendications

1. Utilisation de la 4-(4-bromo-2-fluoroanilino)-6-méthoxy-7-(1-méthylpipéridin-4-ylméthoxy)quinazoline ou d'un sel pharmaceutiquement acceptable de celle-ci et d'un agent anti-tumoral au platine dans la fabrication d'un médicament destiné à être utilisé dans la production d'un effet anti-angiogénique et/ou réducteur de la perméabilité vasculaire chez un animal à sang chaud tel que l'homme.

2. Utilisation de la 4-(4-bromo-2-fluoroanilino)-6-méthoxy-7-(1-méthylpipéridin-4-ylméthoxy)quinazoline ou d'un sel pharmaceutiquement acceptable de celle-ci et d'un agent anti-tumoral au platine dans la fabrication d'un médicament destiné à être utilisé dans la production d'un effet anti-cancéreux chez un animal à sang chaud tel que l'homme.

3. Utilisation de la 4-(4-bromo-2-fluoroanilino)-6-méthoxy-7-(1-méthylpipéridin-4-ylméthoxy)quinazoline ou d'un sel pharmaceutiquement acceptable de celle-ci et d'un agent anti-tumoral au platine dans la fabrication d'un médicament destiné à être utilisé dans la production d'un effet anti-tumoral chez un animal à sang chaud tel que l'homme.

4. Utilisation de la 4-(4-bromo-2-fluoroanilino)-6-méthoxy-7-(1-méthylpipéridin-4-ylméthoxy)quinazoline ou d'un sel pharmaceutiquement acceptable de celle-ci et d'un agent anti-tumoral au platine dans la fabrication d'un médicament destiné à être utilisé dans la production d'un effet anti-angiogénique et/ou réducteur de la perméabilité vasculaire chez un animal à sang chaud tel que l'homme sous traitement avec des radiations ionisantes.

5. Utilisation de la 4-(4-bromo-2-fluoroanilino)-6-méthoxy-7-(1-méthylpipéridin-4-ylméthoxy)quinazoline ou d'un sel pharmaceutiquement acceptable de celle-ci et d'un agent anti-tumoral au platine dans la fabrication d'un médicament destiné à être utilisé dans la production d'un effet anti-cancéreux chez un animal à sang chaud tel que l'homme sous traitement avec des radiations ionisantes.

6. Utilisation de la 4-(4-bromo-2-fluoroanilino)-6-méthoxy-7-(1-méthylpipéridin-4-ylméthoxy) quinazoline ou d' un sel pharmaceutiquement acceptable de celle-ci et d'un agent anti-tumoral au platine dans la fabrication d'un médicament destiné à être utilisé dans la production d'un effet anti-tumoral chez un animal à sang chaud tel que l'homme sous traitement avec des radiations ionisantes.

7. Utilisation selon la revendication 2 ou la revendication 5, **caractérisée en ce que** le cancer est le cancer du poumon non à petites cellules (NSCLC) .

8. Utilisation selon la revendication 3 ou la revendication 6, **caractérisée en ce que** la tumeur est une tumeur du côlon (y compris du rectum), du pancréas, de la vessie, du sein, de la prostate, du poumon, de la vulve ou de la peau.

9. Utilisation selon l'une quelconque des revendications 1-8, **caractérisée en ce que** l'agent anti-tumoral au platine est le cisplatine.

10. Utilisation selon l'une quelconque des revendications 1-8, **caractérisée en ce que** l'agent anti-tumoral au platine est le carboplatine.

11. Utilisation selon l'une quelconque des revendications 1-8, **caractérisée en ce que** l'agent anti-tumoral au platine est l'oxaliplatine.

12. Composition pharmaceutique qui comprend la 4-(4-bromo-2-fluoroanilino)-6-méthoxy-7-(1-méthylpipéridin-4-ylméthoxy)quinazoline ou un sel pharmaceutiquement acceptable de celle-ci, et un agent anti-tumoral au platine, en association avec un excipient ou un support pharmaceutiquement acceptable.

13. Kit comprenant la 4-(4-bromo-2-fluoroanilino)-6-méthoxy-7-(1-méthylpipéridin-4-ylméthoxy)quinazoline ou un sel pharmaceutiquement acceptable de celle-ci, et un agent anti-tumoral au platine.
